# EUROPEAN PATENT APPLICATION

(11) **EP 4 807 008 A1**
(43) Date of publication of application: **16.09.2026**
(21) Application number: 25794956.0
(22) Date of filing: 02.04.2025
(51) Int. Cl.: C12N 15/77, C12N 9/10, C12P 13/10

(54) **GLUTAMATE-BASED AMINO ACID-PRODUCING MICROORGANISM, AND METHOD FOR PRODUCING GLUTAMATE-BASED AMINO ACIDS USING SAME**

(30) Priority: 25.04.2024 KR 20240055638
(71) Applicant: CJ Cheiljedang Corporation, Seoul 04560 (KR)
(72) Inventor: HAN, Seunghee, Seoul 04560 (KR); LEE, Ji Yeon, Seoul 04560 (KR); PARK, So-Yeon, Seoul 04560 (KR); PARK, Sung Eun, Seoul 04560 (KR)
(74) Representative: Meissner Bolte Partnerschaft mbB
(86) International application number: PCT/KR2025/004332
(87) International publication number: WO 2025/225913

(57) **Abstract**

The present disclosure relates to a Corynebacterium sp. microorganism for producing glutamate-based amid acids, and a method for producing glutamate-based amino acids using the microorganism.

## Description

### [TECHNICAL FIELD]

### Cross-reference to related application(s)

This disclosure claims a benefit of priority based on Korean Patent Application No. 10-2024-0055638 filed on April 25, 2024, and all contents disclosed in the documents of the corresponding Korean patent applications are incorporated as part of this disclosure.

The present disclosure relates to a microorganism of the genus *Corynebacterium* for producing glutamate-family amino acids and a method for producing glutamate-family amino acids using the same.

### [BACKGROUND ART]

Glutamate is one of the proteinogenic amino acids widely present in plants, animals, and microorganisms, and is metabolized in organs in the body through conversion into L-ornithine, L-citrulline, L-arginine, putrescine, and the like.

Microorganisms belonging to the genus *Corynebacterium*, particularly *Corynebacterium glutamicum*, are Gram-positive microorganisms that have been widely used for production of amino acids. In the production of amino acids, target substance-specific approaches have been employed, including, for example, increasing the expression level of a gene encoding an enzyme involved mainly in biosynthesis of a desired amino acid and deleting a gene that is not required for biosynthesis of the desired amino acid in strains belonging to the genus *Corynebacterium* (US 9,644,009 B2).

As demand for glutamate-family amino acids has increased, there is a growing need for research on methods for efficiently producing glutamate-family amino acids.

### [DISCLOSURE]

### [TECHNICAL PROBLEM]

An embodiment of the present disclosure provides a microorganism expressing a bifunctional glutamate N-acetyltransferase/amino-acid acetyltransferase (ArgJ) derived from *Thermotoga maritima*.

Another embodiment of the present disclosure provides a composition for producing glutamate-family amino acids comprising a microorganism expressing a bifunctional glutamate N-acetyltransferase/amino-acid acetyltransferase derived from *Thermotoga maritima*.

Another embodiment of the present disclosure provides a method for producing glutamate-family amino acids, comprising culturing the microorganism expressing the bifunctional glutamate N-acetyltransferase/amino acid acetyltransferase derived from *Thermotoga maritima*, and
recovering the glutamate-family amino acids from the cultured microorganism, medium, or both.

Another embodiment of the present disclosure is to provide a use of a microorganism expressing a bifunctional glutamate N-acetyltransferase/amino acid acetyltransferase derived from *Thermotoga maritima* for production of glutamate-family amino acids.

### [TECHNICAL SOLUTION]

In the present disclosure, in order to increase the ability of production for glutamate-family amino acids in a microorganism of the genus *Corynebacterium*, foreign proteins were searched and introduced into the microorganism of the genus *Corynebacterium* to improve the strain.

Accordingly, the present disclosure provides a recombinant strain with improved ability of producing glutamate-family amino acids, by searching for an enzyme having a novel exogenous glutamate N-acetyltransferase activity that increases the ability to produce glutamate-family amino acids of a microorganism of the genus *Corynebacterium*, and introducing the same into a strain of producing glutamate-family amino acid.

In the present disclosure, bifunctional glutamate N-acetyltransferase/amino-acid acetyltransferase derived from *Thermotoga maritima* and a gene encoding the same were selected as representative examples of an enzyme having exogenous glutamate N-acetyltransferase activity, and as a result of expressing them in a microorganism producing glutamate-family amino acids, it was found that the ability to produce glutamate-family amino acids was significantly improved compared to a microorganism in which the gene was not expressed and/or a microorganism to which an enzyme having the same activity as the bifunctional glutamate N-acetyltransferase/amino-acid acetyltransferase derived from *Thermotoga neapolitana* and/or derived from *Corynebacterium glutamicum* was additionally introduced (or enhanced).

An embodiment of the present disclosure provides a microorganism expressing a bifunctional glutamate N-acetyltransferase/amino-acid acetyltransferase (ArgJ) derived from *Thermotoga maritima*.

In one example, the bifunctional glutamate N-acetyltransferase/amino-acid acetyltransferase derived from *Thermotoga maritima* may be, for example, a protein comprising an amino acid sequence of SEQ ID NO: 1, or a protein having sequence identity or homology of 87.5% or more, 88% or more, 89% or more, 90% or more, 91% or more, 92% or more, 93% or more, 94% or more, 95% or more, 96% or more, 97% or more, 98% or more, 99% or more, 99.5% or more, or 99.9% or more with the amino acid sequence of SEQ ID NO: 1.

The microorganism may be one including or expressing the bifunctional glutamate N-acetyltransferase/amino-acid acetyltransferase derived from *Thermotoga maritima*. The microorganism expressing the bifunctional glutamate N-acetyltransferase/amino-acid acetyltransferase derived from *Thermotoga maritima* may be a recombinant microorganism into which a polynucleotide encoding the bifunctional glutamate N-acetyltransferase/aminoacid acetyltransferase derived from *Thermotoga maritima* is introduced. In one example, the polynucleotide encoding the bifunctional glutamate N-acetyltransferase/amino-acid acetyltransferase of SEQ ID NO: 1 may be represented by the nucleotide sequence of SEQ ID NO: 2 or a nucleotide sequence having sequence identity or homology of 87.5% or more, 88% or more, 89% or more, 90% or more, 91% or more, 92% or more, 93% or more, 94% or more, 95% or more, 96% or more, 97% or more, 98% or more, 99% or more, 99.5% or more, or 99.9% or more with the nucleotide sequence of SEQ ID NO: 2.

In the above, the term "homology" means a degree of consistency with a given amino acid sequence or nucleotide sequence and may be expressed as a percentage. In the present disclosure, a homologous sequence thereof having the same or similar activity to a given amino acid sequence or nucleotide sequence is represented as "% homology." For example, it can be confirmed by using standard software for calculating parameters such as score, identity, and similarity, specifically BLAST 2.0, or by comparing sequences by Southern hybridization experiment under defined stringent conditions, and appropriate hybridization conditions to be defined are within the technical scope and can be determined by methods well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

In the present disclosure, the term "bifunctional glutamate N-acetyltransferase/aminoacid acetyltransferase ArgJ" means an enzyme having both glutamate N-acetyltransferase activity of converting glutamate and acetyl-CoA into N-acetylglutamate and amino acid acetyltransferase activity of converting acetyl-L-ornithine and L-glutamate into L-ornithine and N-acetyl-L-glutamate. The bifunctional glutamate N-acetyltransferase/amino-acid acetyltransferase of the present disclosure may be used interchangeably with bifunctional arginine biosynthesis protein ArgJ(Arginine biosynthesis bifunctional protein ArgJ) and ArgJ protein. The "glutamate N-acetyltransferase" may be used interchangeably with terms such as N2-acetyl-L-ornithine: L-glutamate N-acetyltransferase, ornithine acetyltransferase, ornithine transacetylase, and acetylornithine glutamate acetyltransferase, and the "amino acid acetyltransferase" may be used interchangeably with acetyl-CoA:L-glutamate N-acetyltransferase and N-acetylglutamate synthetase. In the present disclosure, the sequence of the bifunctional glutamate N-acetyltransferase/amino-acid acetyltransferase can be obtained from GenBank of NCBI, a known database (e.g., AKE31408.1). Specifically, it may be a polypeptide having bifunctional glutamate N-acetyltransferase/amino-acid acetyltransferase activity encoded by the argJ gene, but is not limited thereto.

The microorganism expressing the bifunctional glutamate N-acetyltransferase/aminoacid acetyltransferase derived from *Thermotoga maritima* may be a glutamate-family amino acid-producing microorganism having an ability to produce glutamate-family amino acids.

In the present disclosure, the term "glutamate-family amino acid-producing microorganism" may be used to mean a case where a microorganism having an ability to produce glutamate-family amino acids is mutated to express the bifunctional glutamate N-acetyltransferase/amino-acid acetyltransferase derived from *Thermotoga maritima* as described above, so that the microorganism has an increased ability to produce glutamate-family amino acids, and/or a case where a microorganism not having an ability to produce glutamate-family amino acids is mutated to express the bifunctional glutamate N-acetyltransferase/amino-acid acetyltransferase derived from *Thermotoga maritima*, so that the microorganism comes to have an ability to produce glutamate-family amino acids. In the present disclosure, "microorganism" encompasses unicellular bacteria and may be used interchangeably with "cell." In the present disclosure, the microorganism before being mutated to express the bifunctional glutamate N-acetyltransferase/amino-acid acetyltransferase derived from *Thermotoga maritima* may be represented as a "parent microorganism or parent strain" or a "host cell" to distinguish it from the mutated microorganism.

In the present disclosure, the term "glutamate-family amino acid" means an amino acid that may be biosynthesized using glutamate as a precursor. Specifically, the glutamate-family amino acid may be any one selected from the group consisting of L-ornithine, L-citrulline, L-arginine, and putrescine, but is not limited thereto, as long as it is an amino acid that may be biosynthesized using glutamate as a precursor. The "glutamate-family amino acid" of the present disclosure may be used interchangeably with "glutamic acid-family amino acid."

In one example, the microorganism may be one selected from all microorganisms having an ability to produce glutamate-family amino acids. In one example, the microorganism, for example, the parent microorganism or parent strain before mutation, may be (1) a microorganism naturally having an ability to produce glutamate-family amino acids, or (2) a microorganism having an ability to produce glutamate-family amino acids or having an improved ability to produce glutamate-family amino acids by introducing a mutation into the microorganism naturally having an ability to produce glutamate-family amino acids or a strain having no or significantly low ability to produce glutamate-family amino acids.

In one embodiment, the microorganism may be one or more selected from the group consisting of (1) a microorganism naturally having an ability to produce glutamate-family amino acids, or (2) all microorganisms of the genus *Corynebacterium* having an ability to produce glutamate-family amino acids or having an improved ability to produce glutamate-family amino acids by introducing a mutation into the microorganism naturally having an ability to produce glutamate-family amino acids, or the parent microorganism or parent strain having no or significantly low ability to produce glutamate-family amino acids.

The microorganism of the genus *Corynebacterium* may include *Corynebacterium glutamicum*, *Corynebacterium stationis, Brevibacterium lactofermentum, Brevibacterium flavum, Corynebacterium thermoaminogenes, Corynebacterium efficiens*, and the like, but is not necessarily limited thereto. More specifically, the microorganism of the genus *Corynebacterium* may be *Corynebacterium glutamicum*.

In one example, the microorganism expressing the bifunctional glutamate N-acetyltransferase/amino-acid acetyltransferase derived from *Thermotoga maritima* may be one in which a mutation for expressing the bifunctional glutamate N-acetyltransferase/amino-acid acetyltransferase derived from *Thermotoga maritima* is introduced, so that the ability to produce glutamate-family amino acids is increased compared to a homologous unmodified microorganism and/or a microorganism expressing an enzyme having glutamate N-acetyltransferase activity derived from a microorganism of another genus or another species.

The unmodified microorganism is a microorganism that does not express the bifunctional glutamate N-acetyltransferase/amino-acid acetyltransferase derived from *Thermotoga maritima*, and may mean a homologous microorganism into which a mutation for expressing the bifunctional glutamate N-acetyltransferase/amino-acid acetyltransferase derived from *Thermotoga maritima* is not introduced or a microorganism before the mutation is introduced.

The enzyme having the glutamate N-acetyltransferase activity derived from a microorganism of another genus or another species other than the *Thermotoga maritima* may be a bifunctional glutamate N-acetyltransferase/amino-acid acetyltransferase derived from a microorganism of the genus Thermotoga excluding *Thermotoga maritima* and/or derived from a microorganism of the genus *Corynebacterium*, and specifically may be bifunctional glutamate N-acetyltransferase/amino-acid acetyltransferase derived from *Thermotoga neapolitana* (represented by an amino acid sequence of SEQ ID NO: 4) or bifunctional glutamate N-acetyltransferase/amino acid acetyltransferase derived from *Corynebacterium glutamicum* (represented by an amino acid sequence of SEQ ID NO: 7), but is not limited thereto.

In the present disclosure, the "mutation for expressing a bifunctional glutamate N-acetyltransferase/amino-acid acetyltransferase derived from *Thermotoga maritima*" may mean all manipulations for expressing a bifunctional glutamate N-acetyltransferase/amino-acid acetyltransferase derived from *Thermotoga maritima* described above in the parent strain. In one example, the mutation for expressing the bifunctional glutamate N-acetyltransferase/amino-acid acetyltransferase derived from *Thermotoga maritima* may be introducing a polynucleotide encoding the bifunctional glutamate N-acetyltransferase/aminoacid acetyltransferase derived from *Thermotoga maritima* or a recombinant vector including the same into the parent strain.

The "microorganism into which a mutation for expressing a bifunctional glutamate N-acetyltransferase/amino-acid acetyltransferase derived from *Thermotoga maritima* is introduced" or "microorganism mutated to express the bifunctional glutamate N-acetyltransferase/amino-acid acetyltransferase derived from *Thermotoga maritima*" may be a microorganism into which a polynucleotide encoding the bifunctional glutamate N-acetyltransferase/amino-acid acetyltransferase derived from *Thermotoga maritima* or a recombinant vector including the same is introduced, and may be one in which an ability to produce glutamate-family amino acids is imparted or increased compared to an unmodified microorganism and/or a microorganism expressing an enzyme having glutamate N-acetyltransferase activity derived from a microorganism of another genus or another species.

In one example, the parent strain may be a wild-type, or one mutated to increase the ability to produce glutamate-family amino acids, for example, one in which the activity of a protein involved in the biosynthesis or metabolism of glutamate-family amino acids is regulated (increased (promoted) or decreased (inhibited)) compared to a wild-type, but is not limited thereto. For example, the parent strain may be a wild-type *Corynebacterium glutamicum* strain, a *Corynebacterium glutamicum* ATCC13869 strain in which the activities of an arginine repressor (ArgR) and/or an ornithine carbamoyltransferase (ArgF) are attenuated, a *Corynebacterium glutamicum* ATCC13869 strain in which the activities of an arginine repressor (ArgR) and/or an argininosuccinate synthase (ArgG) are attenuated, or a *Corynebacterium glutamicum* strain in which the activities of an argininosuccinate synthase (ArgG) and/or an ornithine carbamoyltransferase (ArgF) are attenuated and the activity of an ornithine decarboxylase (ODC) is introduced, but is not limited thereto.

In another example of the present disclosure, the microorganism of the genus *Corynebacterium* with increased ability to produce glutamate-family amino acids of the present disclosure may be a microorganism in which the ability to produce glutamate-family amino acids is strengthened by additionally strengthening the activity of a part of proteins in an L-amino acid biosynthetic pathway or by additionally attenuating the activity of a part of proteins in an L-amino acid degradation pathway.

Specifically, the microorganism of the genus *Corynebacterium* of the present disclosure may be a strain in which the activity of an ornithine carbamoyltransferase subunit F (ArgF) and/or an arginine repressor (ArgR) is additionally attenuated, or an argF gene and/or an argR gene encoding them are further deleted. In addition, the strain of the present disclosure may be a strain mutated so that the activities of an arginine repressor (ArgR) and/or an argininosuccinate synthase (ArgG) are additionally attenuated, or an argR gene and/or an argG gene encoding them are additionally deleted and/or the activity of an ornithine decarboxylase (ODC) is introduced.

The amino acid sequences of the ArgF, ArgR, ArgG, and ODC can be obtained from GenBank of NCBI, which is a known database. As an example, the amino acid sequence of ArgF of the present disclosure may comprise ANU33618.1 derived from *Corynebacterium glutamicum* ATCC13869 or an amino acid sequence having homology of 90% or more therewith, the amino acid sequence of ArgR may comprise ANU33619.1 derived from *Corynebacterium glutamicum* ATCC13869 or an amino acid sequence having homology of 90% or more therewith, the amino acid sequence of ArgG may comprise ANU33620.1 derived from *Corynebacterium glutamicum* ATCC13869 or an amino acid sequence having homology of 90% or more therewith, and the amino acid sequence of ODC may comprise AAA64830.1 derived from Lactobacillus sp. 30A strain or an amino acid sequence having homology of 90% or more therewith, but it is not limited thereto, and it is obvious to comprise proteins having ArgF, ArgR, ArgG, or ODC activity derived from various sources.

The term "introduction of activity" in the present invention may mean that the activity of a protein that is not present or is insufficient in a microorganism is newly introduced or increased in the microorganism, and specifically, may include inserting or delivering a gene encoding a protein that is not exist in the microorganism into the microorganism in an expressible manner, or inducing a mutation to strengthen the expression of a protein that is not expressed or is hardly expressed in the microorganism, but is not limited to the above examples.

Meanwhile, in the present invention, mutations such as introduction of activity, strengthening of activity, or attenuation of activity may occur through a process called transformation, and the term "transformation" in the present invention means introducing a polynucleotide encoding a specific protein or a vector including a promoter sequence with strong or weak activity into a host cell so that the protein encoded by the polynucleotide can be expressed in the host cell or inducing a mutation in the chromosome of the host cell. In addition, the polynucleotide includes DNA and RNA encoding a target protein. The polynucleotide may be introduced in any form as long as it can be introduced into the host cell to induce expression or mutation. For example, the polynucleotide may be introduced into the host cell in the form of an expression cassette, which is a genetic construct including all elements necessary for its own expression. The expression cassette may typically include a promoter, a transcription termination signal, a ribosome-binding site, and a translation termination signal operably linked to the polynucleotide. The expression cassette may be in the form of an expression vector capable of self-replication. In addition, the polynucleotide may be introduced into the host cell in its own form and operably linked to a sequence required for expression in the host cell, but is not limited thereto.

In addition, in the above, the term "operably linked" means that a promoter sequence for initiating and mediating transcription of a polynucleotide encoding a specific protein of the present invention and the gene sequence are functionally connected.

The term "vector" used in the present invention refers to a DNA construct containing the nucleotide sequence of a polynucleotide encoding a target protein operably linked to a suitable regulatory sequence so as to express the target protein in a suitable host. The regulatory sequence includes a promoter capable of initiating transcription, any operator sequence for regulating such transcription, a sequence encoding a suitable mRNA ribosome-binding site, and a sequence regulating the termination of transcription and translation. The vector may be replicated or function independently of the host genome after being transformed into a suitable host cell, and may be integrated into the genome itself.

The vector used in the present invention is not particularly limited as long as it may be replicable in the host cell, and any vector known in the art may be used. Examples of commonly used vectors include plasmids, cosmids, viruses, and bacteriophages in a natural or recombinant state. For example, pWE15, M13, MBL3, MBL4, IXII, ASHII, APII, t10, t11, Charon4A, Charon21A, etc., may be used as phage vectors or cosmid vectors, and pBR-based, pUC-based, pBluescriptII-based, pGEM-based, pTZ-based, pCL-based, and pET-based, etc., may be used as plasmid vectors. The vector being usable in the present invention is not particularly limited, and known expression vectors can be used. Specifically, pDZ, pDZTn, pACYC177, pACYC184, pCL, pECCG117, pUC19, pBR322, pMW118, pCC1BAC, pDCM2, pDC24 vectors, etc., can be used.

In the present disclosure, the term "attenuation" of the activity of a polypeptide is a concept including both a case where the activity is reduced compared to the intrinsic activity and a case where there is no activity. The attenuation may be used interchangeably with the terms such as inactivation, deficiency, down-regulation, decrease, reduce, and attenuation.

The attenuation may also include cases in that the activity of the polypeptide itself is reduced or removed compared to the activity of the polypeptide originally possessed by the microorganism due to a mutation of a polynucleotide encoding the polypeptide, cases in that the overall level of polypeptide activity and/or concentration (expression level) in the cell is lower than that of the original strain due to inhibition of expression of the polynucleotide encoding the same or inhibition of translation into the polypeptide, cases in that the polynucleotide is not expressed at all, and/or cases in that there is no activity of the polypeptide even if the polynucleotide is expressed. The " intrinsic activity" means an activity of a specific polypeptide originally possessed by a parent strain, a wild-type, or an unmodified microorganism before the characteristic is changed when the characteristic is changed due to a genetic mutation by a natural or artificial factor. This may be used interchangeably with "activity before modification." That the activity of a polypeptide is "inactivated, deficient, decreased, down-regulated, reduced, or attenuated" compared to the intrinsic activity means that it is lower than the activity of the specific polypeptide originally possessed by the parent strain or unmodified microorganism before the change of characteristic.

Such attenuation of the polypeptide activity may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the attenuation of the polypeptide in the present disclosure may include:
1) deletion of all or part of a gene encoding a polypeptide;
2) modification of an expression-regulating region (or expression-regulating sequence) to reduce expression of a gene encoding a polypeptide;
3) modification of an amino acid sequence constituting the polypeptide to eliminate or attenuate an activity of a polypeptide (e.g., deletion/substitution/addition of one or more amino acids in an amino acid sequence);
4) modification of a gene sequence encoding a polypeptide to eliminate or attenuate an activity of a polypeptide (for example, deletion/substitution/addition of one or more nucleotides in a nucleotide sequence of a gene to encode the polypeptide modified to eliminate or attenuate the activity of the polypeptide);
5) modification of a nucleotide sequence encoding a start codon or a 5'-UTR region of a gene transcript encoding a polypeptide;
6) introduction of an antisense oligonucleotide (e.g., antisense RNA) that binds complementarily to a gene transcript encoding a polypeptide;
7) addition of a sequence complementary to Shine-Dalgarno sequence to the front end of the Shine-Dalgarno sequence of a gene encoding a polypeptide to form a secondary structure to which ribosomal cannot attach;
8) addition of a promoter that is transcribed in the opposite direction to the 3' end of an open reading frame (ORF) of a gene sequence encoding a polypeptide (Reverse Transcription Engineering, RTE); or
9) regulation of intracellular localization of a polypeptide; or
10) a combination of two or more selected from the above 1) to 9), but is not particularly limited thereto.

For example,
The 1) deletion of part or all of the gene encoding a polypeptide may be the removal of the entire polynucleotide encoding the endogenous target polypeptide within the chromosome, replacement with a polynucleotide in which some nucleotides are deleted, or replacement with a marker gene.

In addition, the 2) modification of an expression-regulating region (or expression-regulating sequence) may be a deletion, insertion, non-conservative or conservative substitution, or a combination thereof, resulting in a mutation on the expression regulatory region (or expression regulatory sequence), or a replacement with a sequence having weaker activity. The expression-regulating region includes, but is not limited to, a promoter, an operator sequence, a sequence coding for a ribosome binding site, and a sequence regulating the termination of transcription and translation.

In addition, the 3) modification of a nucleotide sequence encoding a start codon or a 5'-UTR region of a gene transcript encoding the polypeptide may be, for example, substitution with a nucleotide sequence coding for another start codon that has a lower polypeptide expression rate compared to the intrinsic start codon, but is not limited thereto.

In addition, the modification of an amino acid sequence or polynucleotide sequence in 4) and 5) may be a sequence variation by deletion, insertion, non-conservative or conservative substitution, or a combination thereof, of the amino acid sequence of the polypeptide or the polynucleotide sequence encoding the polypeptide to weaken the activity of the polypeptide, or a replacement with an amino acid sequence or polynucleotide sequence modified to have weaker activity or an amino acid sequence or polynucleotide sequence modified to have no activity, but is not limited thereto. For example, gene expression may be inhibited or weakened by introducing a variation within the polynucleotide sequence to form a stop codon, but is not limited thereto.

The 6) introduction of an antisense oligonucleotide (e.g., antisense RNA) that binds complementarily to the transcript of the gene encoding the polypeptide above, refer, for example, to the literature [Weintraub, H. et al., Antisense-RNA as a molecular tool for genetic analysis, Reviews - Trends in Genetics, Vol. 1(1) 1986].

The 7) addition of a sequence complementary to a Shine-Dalgarno sequence to the front end of the Shine-Dalgarno sequence of a gene encoding a polypeptide to form a secondary structure that makes attachment of a ribosome impossible may make mRNA translation impossible or reduce the rate.

The 8) addition of a promoter that is transcribed in the opposite direction to the 3' end of an open reading frame (ORF) of a gene sequence encoding the polypeptide (Reverse transcription engineering, RTE) may weaken the activity by making an antisense nucleotide complementary to the transcript of the gene encoding the polypeptide.

The 9) regulation of intracellular localization of a polypeptide may be targeting the polypeptide to a specific organelle or a specific intracellular space. For example, it may be targeting the polypeptide to a specific intracellular organelle or a specific intracellular space. For example, it may involve targeting to the periplasm or cytoplasm through the addition or removal of a leader sequence that functions for the targeting of the polypeptide, but is not limited thereto.

Such attenuation of polypeptide activity may involve a reduction in the activity or concentration expression of the corresponding polypeptide relative to the activity or concentration of the polypeptide expressed in the wild type or the microbial strain prior to modification, or an increase in the amount of products produced from the polypeptide, but is not limited thereto.

In the present disclosure, the term "enhancement" of polypeptide activity means that the activity of the polypeptide is increased compared to the intrinsic activity. The enhancement may be used interchangeably with terms such as activation, up-regulation, overexpression, and increase. Here, activation, enhancement, up-regulation, overexpression, and increase may include both exhibiting activity that was not originally possessed, or exhibiting improved activity compared to the intrinsic activity or activity prior to modification. The "intrinsic activity" refers to the activity of a specific polypeptide originally possessed by a parent strain or an unmodified microorganism before the characteristic is changed when the characteristic is changed due to a genetic mutation by a natural or artificial factor. This may be used interchangeably with "activity before modification." That the activity of a polypeptide is "enhanced," "up-regulated," "overexpressed," or "increased" compared to the intrinsic activity means that it has been enhanced compared to the activity and/or concentration (expression amount) of a specific polypeptide originally possessed by the parent strain or non-modified microorganism before the characteristic is changed.

The enhancement can be achieved by introducing a exogenous polypeptide or enhancing activity and/or concentration (expression level) of an endogenous polypeptide. Whether the activity of the polypeptide is enhanced can be confirmed by an increase in the degree of activity, expression level, or amount of products released from the polypeptide.

The enhancement of the polypeptide activity can be applied by applying various methods well known in the art, and is not limited to those that can enhance the activity of the target polypeptide compared to the microorganism before modification. Specifically, it may utilize, but is not limited to, genetic engineering and/or protein engineering known to a person skilled in the art, which are routine methods of molecular biology (e.g., Sitnicka et al. Functional Analysis of Genes. Advances in Cell Biology. 2010, Vol. 2. 1-16; Sambrook et al. Molecular Cloning 2012, etc.).

Specifically, the enhancement of the polypeptide of the present disclosure may include:
1) increase in the intracellular copy number of a polynucleotide encoding a polypeptide;
2) replacement of a chromosomal gene expression-regulating region in a gene encoding a polypeptide with a sequence having strong activity;
3) modification of a nucleotide sequence encoding a start codon or a 5'-UTR region of a gene transcript encoding a polypeptide;
4) modification of an amino acid sequence of the polypeptide to enhance polypeptide activity;
5) modification of a polynucleotide sequence encoding the polypeptide to enhance polypeptide activity (for example, modification of a polynucleotide sequence of a gene, to encode the polypeptide modified to enhance the polypeptide activity);
6) introduction of an exogenous polypeptide exhibiting polypeptide activity or a exogenous polynucleotide encoding the same;
7) codon optimization of a polynucleotide encoding a polypeptide;
8) selection, and modification or chemical modification of an exposed site by analyzing the tertiary structure of a polypeptide; or
9) regulation of intracellular localization of a polypeptide; or
10) a combination of two or more selected from the above 1) to 9), but is not particularly limited thereto.

More specifically,
The 1) increase in the intracellular copy number of a polynucleotide encoding a polypeptide may be achieved by introducing into a host cell a vector to which the polynucleotide encoding the polypeptide is operably linked, which can replicate and function independently of the host. Alternatively, it may be achieved by introducing one or more copies of the polynucleotide encoding the polypeptide into the chromosome in the host cell. The introduction into the chromosome may be performed by introducing into the host cell a vector capable of inserting the polynucleotide into the chromosome in the host cell, but is not limited thereto. The vector is as described above.

The 2) replacement of a chromosomal gene expression-regulating region in a gene encoding a polypeptide with a sequence having strong activity may be, for example, involve deletion, insertion, non-conservative or conservative substitution, or a combination thereof, thereby causing a sequence mutation to further enhance the activity of the expression regulatory region, or replacement with a sequence having greater activity. The expression-regulating region may include, but is not limited to, a promoter, an operator sequence, a sequence encoding a ribosome binding site, and a sequence regulating the termination of transcription and translation. As an example, the original promoter may be replaced with a strong promoter, but is not limited thereto.

Examples of known strong promoters include CJ1 to CJ7 promoters (U.S. Patent No. 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent No. 10584338 B2), O2 promoter (U.S. Patent No. 10273491 B2), tkt promoter, yccA promoter, etc., but are not limited thereto.

The 3) modification of a nucleotide sequence encoding a start codon or a 5'-UTR region of a gene transcript encoding a polypeptide may be, for example, a substitution with a nucleotide sequence encoding another start codon that has a higher polypeptide expression rate compared to the intrinsic start codon, but is not limited thereto.

The modification of the amino acid sequence or polynucleotide sequence in 4) and 5) may be involve the occurrence of sequence mutations in the amino acid sequence of the polypeptide or the polynucleotide sequence encoding the polypeptide by deletion, insertion, non-conservative or conservative substitution, or a combination thereof, to enhance the activity of the polypeptide, or may involve replacement with an amino acid sequence or polynucleotide sequence modified to have stronger activity or an amino acid sequence or polynucleotide sequence modified to increase activity, but is not limited thereto. The replacement may be performed by inserting the polynucleotide into the chromosome by homologous recombination, but is not limited thereto. The vector used in this case may additionally include a selection marker to confirm whether chromosome insertion has occurred. The selection marker is as described above.

The 6) introduction of an exogenous polypeptide exhibiting polypeptide activity may be introduction into a host cell of a exogenous polynucleotide encoding a polypeptide exhibiting the same/similar activity as the polypeptide. The exogenous polynucleotide is not limited in its origin or sequence as long as it exhibits the same/similar activity as the polypeptide. The method used for the introduction may be performed by a person skilled in the art by appropriately selecting a known transformation method, and the polypeptide may be generated and its activity increased by the expression of the introduced polynucleotide within the host cell.

The 7) codon optimization of a polynucleotide encoding a polypeptide may be codon optimization of an endogenous polynucleotide to increase transcription or translation in a host cell, or codon optimization of a exogenous polynucleotide so that optimized transcription and translation occurs within the host cell.

The 8) selection, and modification or chemical modification of an exposed site by analyzing the tertiary structure of a polypeptide may be, for example, determining a template protein candidate based on the degree of sequence similarity by comparing sequence information of the polypeptide to be analyzed with a database in which sequence information of known proteins is stored, and analyzing structure based on this, to select; and modify or chemically modify an exposed site.

The 9) regulation of intracellular localization of a polypeptide may be targeting the polypeptide to a specific organelle or a specific intracellular space. For example, it may be targeting to the periplasm or cytoplasm through addition or removal of a leader sequence functioning in targeting of a polypeptide, but is not limited thereto.

Such enhancement of polypeptide activity may be increasing the activity or concentration of the corresponding polypeptide based on the activity or concentration of the polypeptide expressed in the wild type or the microbial strain before modification, or increasing the amount of the product produced from the polypeptide, but is not limited thereto.

Modification of part or all of the polynucleotide in the microorganism of the present disclosure may be induced by (a) homologous recombination using a vector for chromosomal insertion in the microorganism or genome editing using an engineered nuclease (e.g., CRISPR-Cas9) and/or (b) treatment with light such as ultraviolet rays and radiation and/or chemicals, but is not limited thereto. The method of modifying part or all of the gene may include methods using DNA recombination technology. For example, deletion of all or part of the gene can be achieved by injecting a nucleotide sequence or vector containing a nucleotide sequence homologous to the target gene into the microorganism to allow homologous recombination to occur. The injected nucleotide sequence or vector may include a dominant selection marker, but is not limited thereto.

Another embodiment of the present disclosure provides a method for increasing the ability of the microorganism of the genus *Corynebacterium* to produce glutamate-family amino acids or a method for conferring the ability to produce glutamate-family amino acids to the microorganism, comprising a step of introducing (transforming) a bifunctional glutamate N-acetyltransferase/amino-acid acetyltransferase derived from *Thermotoga maritima*, a polynucleotide encoding the same, or a recombinant vector containing the polynucleotide into a microorganism of the genus *Corynebacterium*.

The bifunctional glutamate N-acetyltransferase/amino-acid acetyltransferase derived from *Thermotoga maritima*, the polynucleotide, the glutamate-family amino acids, and the microorganism of the genus *Corynebacterium* are as described above.

Another embodiment of the present disclosure provides a method for producing glutamate-family amino acids, comprising the step of culturing the microorganism of the genus *Corynebacterium* expressing the bifunctional glutamate N-acetyltransferase/amino-acid acetyltransferase derived from *Thermotoga maritima* in a medium.

In the present disclosure, the term "culturing" means growing the microorganism of the genus *Corynebacterium* of the present disclosure under appropriately controlled environmental conditions. The culture process of the present disclosure may be performed according to suitable media and culture conditions known in the art. Such culture process can be easily adjusted and used by those skilled in the art depending on the selected strain. Specifically, the culture may be batch, continuous and/or fed-batch, but is not limited thereto.

In the present disclosure, the term "medium" means substances in which nutritional substances required for culturing the microorganism of the genus *Corynebacterium* of the present disclosure are mixed as main components, and supplies nutritional substances and growth factors as well as water which are indispensable for survival and growth. Specifically, the medium and other culture conditions used for culturing the microorganism of the genus *Corynebacterium* of the present disclosure may be any medium used for culturing ordinary microorganisms without special limitations; however, the microorganisms of the genus *Corynebacterium* of this disclosure may be cultured under aerobic conditions while controlling the temperature, pH, etc., in a conventional medium containing a suitable carbon source, nitrogen source, phosphorus, inorganic compounds, amino acids, and/or vitamins.

In the present disclosure, the carbon source may include carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamic acid, methionine, and lysine, and the like. In addition, natural organic nutrient sources such as starch hydrolysate, molasses, blackstrap molasses, rice bran, cassava, sugarcane bagasse, and corn steep liquor can be used, and specifically, carbohydrates such as glucose and sterilized pretreated molasses (i.e., molasses converted to reducing sugar) can be used, and other suitable amounts of carbon sources can be used in various ways without limitation. These carbon sources may be used alone or in combination of two or more, but are not limited thereto.

As the nitrogen source, inorganic nitrogen sources such as ammonia, ammonium sulfate, ammonium chloride, ammonium acetate, ammonium phosphate, ammonium carbonate, and ammonium nitrate; organic nitrogen sources such as amino acids including glutamic acid, methionine, glutamine and the like, peptone, NZ-amine, meat extract, yeast extract, malt extract, corn steep liquor, casein hydrolysate, fish or a decomposition product thereof, and defatted soybean cake or a decomposition product thereof can be used. These nitrogen sources may be used alone or in combination of two or more, but are not limited thereto.

The phosphorus source may include monopotassium phosphate, dipotassium phosphate, or a corresponding sodium-containing salt, and the like. As the inorganic compound, sodium chloride, calcium chloride, iron chloride, magnesium sulfate, iron sulfate, manganese sulfate, calcium carbonate, and the like can be used, and in addition, amino acids, vitamins and/or appropriate precursors can be included. These components or precursors can be added to the medium in a batch or continuous manner. However, it is not limited thereto.

In addition, during culturing the microorganism of the genus *Corynebacterium* of the present disclosure, compounds such as ammonium hydroxide, potassium hydroxide, ammonia, phosphoric acid, and sulfuric acid can be added to the medium in an appropriate manner to adjust the pH of the medium. In addition, during culture an antifoaming agent such as fatty acid polyglycol ester can be used to inhibit foam generation. In addition, to maintain the aerobic state of the medium, oxygen or an oxygen-containing gas may be injected into the medium, or to maintain anaerobic and microaerobic conditions, it may be performed without gas injection or by injecting nitrogen, hydrogen, or carbon dioxide gas, but it is not limited thereto.

In the culture of the present disclosure, the culturing temperature can be maintained at 20 to 45°C, specifically 25 to 40°C, and culture can be performed for about 10 to 160 hours, but it is not limited thereto.

The glutamate-family amino acids produced by the culture of the present disclosure may be secreted into the medium or remain in the cells.

In the method for producing glutamate-family amino acids of the present disclosure, the glutamate-family amino acids may be at least one selected from the group consisting of L-ornithine, L-citrulline, L-arginine, and putrescine.

The method for producing glutamate-family amino acids of the present disclosure may further comprise, for example, before the culturing step, a step of preparing the microorganism of the genus *Corynebacterium* of the present disclosure, a step of preparing a medium for culturing the microorganism, or a combination thereof (in any order).

The method for producing glutamate-family amino acids of the present disclosure may further comprise a step of recovering the glutamate-family amino acids from the medium according to the culture (medium in which culture has been performed) or the microorganism of the genus *Corynebacterium*. The recovering step may be additionally included after the culturing step.

The recovery may be collecting desired glutamate-family amino acids using a suitable method known in the art according to the cultivation method of the microorganism of the present disclosure, for example, batch, continuous, or fed-batch culture method. For example, centrifugation, filtration, treatment with a crystallization protein precipitant (salting-out method), extraction, ultrasonic disruption, ultrafiltration, dialysis, various chromatographies such as molecular sieve chromatography (gel filtration), adsorption chromatography, ionexchange chromatography, affinity chromatography, HPLC, or a combination of these methods can be used, and desired glutamate-family amino acids can be recovered from the medium or microorganism using a suitable method known in the art.

In addition, the method for producing glutamate-family amino acids of the present disclosure may additionally comprise a purification step. The purification can be performed using a suitable method known in the art. In one example, when the method for producing glutamate-family amino acids of the present disclosure comprises both a recovery step and a purification step, the recovery step and the purification step may be performed continuously or discontinuously regardless of the order, or may be performed simultaneously or integrated into one step, but it is not limited thereto.

In the method of the present disclosure, the bifunctional glutamate N-acetyltransferase/amino-acid acetyltransferase derived from *Thermotoga maritima*, the glutamate-family amino acid, and the microorganism of the genus *Corynebacterium*, etc., are as described in the other examples above.

Another embodiment of the present disclosure provides a composition for producing glutamate-family amino acids comprising a microorganism expressing the bifunctional glutamate N-acetyltransferase/amino-acid acetyltransferase derived from *Thermotoga maritima*.

The bifunctional glutamate N-acetyltransferase/amino-acid acetyltransferase derived from *Thermotoga maritima*, the glutamate-family amino acid, and the microorganism, etc., are as described in the other examples above.

The composition may further comprise any suitable excipient commonly used in a composition for production of a target product, and such an excipient may be, for example, a preservative, a wetting agent, a dispersant, a suspending agent, a buffer, a stabilizer, or an isotonic agent, and the like, but is not limited thereto.

Another embodiment provides a use of a microorganism in the production of glutamate-family amino acids.

Another embodiment provides a use of a microorganism in the manufacture of a composition for producing glutamate-family amino acids.

Another embodiment of the present disclosure provides a microorganism, method, composition, product, process, or use characterized by one or more elements disclosed in the present disclosure.

### [ADVANTAGEOUS EFFECTS]

The microorganism of the genus *Corynebacterium* expressing a bifunctional glutamate N-acetyltransferase/amino-acid acetyltransferase derived from *Thermotoga maritima* of the present disclosure has been demonstrated to have an increased ability of producing glutamate-family amino acids compared to a parent strain and/or a microorganism of the genus *Corynebacterium* expressing an enzyme having glutamate N-acetyltransferase activity derived from another species of microorganism, and thus can be widely utilized for the production of glutamate-family amino acids.

### [MODE FOR INVENTION]

Hereinafter, the present disclosure will be described in more detail by examples. However, the following examples are merely preferred embodiments for illustrating the present disclosure, and therefore, it is not intended to limit the scope of rights of the present disclosure thereto. Meanwhile, technical matters not described in the present disclosure can be sufficiently understood and easily implemented by those of ordinary skill in the technical field of the present disclosure or similar technical fields.

### Example 1. Construction of expression vector introduced with the bifunctional glutamate N-acetyltransferase/amino acid acetyltransferase gene derived from a heat-resistant microorganism

In order to evaluate the effect of introducing a bifunctional glutamate N-acetyltransferase/amino acid acetyltransferase derived from the thermophilic microorganism on the production of glutamate-family amino acids, an expression vector into which two types of bifunctional glutamate N-acetyltransferase/amino-acid acetyltransferase derived from a heat-resistant microorganism were introduced was constructed as follows.

Specifically, information on a gene encoding bifunctional glutamate N-acetyltransferase/amino-acid acetyltransferase derived from *Thermotoga maritima* (SEQ ID NO: 2) and surrounding nucleotide sequences and information on a gene encoding bifunctional glutamate N-acetyltransferase/amino-acid acetyltransferase derived from *Thermotoga neapolitana* (SEQ ID NO: 5) and surrounding nucleotide sequences were obtained from NIH GenBank.

Gene fragments for constructing vectors were obtained through PCR using sequences obtained through gene synthesis as templates based on the sequences obtained above. At this time, SolgTM Pfu-X DNA polymerase was used as a polymerase, and the PCR conditions were as follows: initial denaturation at 95 °C for 10 minutes, followed by 30 cycles of denaturation at 95 °C for 30 seconds, annealing at 55 °C for 30 seconds, and extension at 72 °C for 1 minute, with a final extension performed at 72 °C for 5 minutes.

More specifically, primers of SEQ ID NOs. 9 and 10 were prepared to amplify a gene derived from *Thermotoga neapolitana*, and PCR was performed using SEQ ID NO. 6 as a template, resulting in a gene fragment of 1194 bp. Primers of SEQ ID NOs. 11 and 12 were prepared to amplify a gene derived from *Thermotoga maritima*, and PCR was performed using SEQ ID NO. 3 as a template, resulting in a gene fragment of 1194 bp.

To amplify a gene derived from *Corynebacterium glutamicum* ATCC13869 (SEQ ID NO: 8) as a control,, primers of SEQ ID NO: 13 and 14 were prepared using the genome of wild-type *Corynebacterium glutamicum* ATCC 13869 (NZ_CP016335.1) as a template, and as a result of performing PCR, a gene fragment of 1167 bp was obtained. In order to obtain an expression promoter in the vector, PCR was performed in the same manner as above using a gene upstream of NCgl0856 of wild-type *Corynebacterium glutamicum* ATCC 13869 as a template and primers of SEQ ID NO: 15 and SEQ ID NO: 16, to obtain a gene fragment of promoter region of 500 bp.

The gene fragment of promoter region and the gene fragments derived from heat-resistant microorganisms obtained above were fusion-cloned into a pCES208 ("Construction of heat-inducible expression vector of Corynebacterium glutamicum and C. ammoniagenes: fusion of lambda operator with promoters isolated from C. ammoniagenes." Journal of microbiology and biotechnology 18.4 (2008): 639-647.) vector cleaved with BamHI and XbaI restriction enzymes using an In-fusion Cloning Kit to obtain each gene expression vector. The vector including the gene fragment derived from *Thermotoga neapolitana* and the promoter was named "pCES208-PbetP-arg(T.ne)", the vector including the gene fragment derived from *Thermotoga maritima* and the promoter was named "pCES208-PbetP-argJ(T.ma)", and the vector including the gene fragment derived from *Corynebacterium glutamicum* ATCC13869 and the promoter was named "pCES208-PbetP-argJ(C.gl)".

The sequences of the primers used in Example 1 are shown in Table 1 below.

**[Table 1]**

| Name | Sequence (5'-> 3') | SEQ ID NO |
|---|---|---|
| TnJ_F | CAAATTCGAAACCGATAATGTTCGTCCC TAGGGG | 9 |
| TnJ_R | GTGGCGGCCGCTCTAGATTAGGTGCGG TAGCGGC | 10 |
| TmJ_F | CAAATTCGAAACCGATAATGTTCACCCC ACGCGG | 11 |
| TmJ_R | GTGGCGGCCGCTCTAGATTAGGTGCGAT AGCGAC | 12 |
| CgJ_F | CAAATTCGAAACCGATAATGGCCAAAA AAGGCAT | 13 |
| CgJ_R | GTGGCGGCCGCTCTAGATTAAGAGCTG TACGCGG | 14 |
| PbetP_F | TGCAGCCCGGGGGATCCCAGCCGAAGT TTTAGGT | 15 |
| PbetP_R | TATCGGTTTCGAATTTGGGTCAGATGTA GTCATA | 16 |

### Example 2. Evaluation of L-ornithine production ability of a microorganism introduced with the bifunctional glutamate N-acetyltransferase/amino acid acetyltransferase gene derived from a heat-resistant microorganism

### 2-1. Construction of a microorganism producing L-ornithine

In order to construct an L-ornithine-producing microorganism, a vector was constructed to replace glutamic acid at position 47 of the amino acid sequence of ArgR (ANU33619.1) with a stop codon.

By using the genome of wild-type *Corynebacterium glutamicum* ATCC13869 as a template, a homologous recombinant A arm was amplified using the primer pair of SEQ ID NOs: 17 and 18, and a homologous recombinant B arm was amplified using the primer pair of SEQ ID NOs: 19 and 20. At this time, PCR conditions were as follows: initial denaturation at 95 °C for 10 minutes, followed by 30 cycles of denaturation at 95 °C for 30 seconds, annealing at 55 °C for 30 seconds, and extension at 72°C for 1 minute, with a final extension performed at 72 °C for 5 minutes. A plasmid was obtained by cloning the amplified homologous recombinant arms A and B and the vector pDC24 (SEQ ID NO: 40) cleaved with BamHI and XbaI restriction enzymes. This plasmid was named pDC24-argR(E47*).

In order to produce a microorganism with further improved productivity for L-ornithine, a vector was constructed to replace serine at position 55 of the amino acid sequence of protein ArgF (ANU33618.1) with a stop codon. By using the genome of *Corynebacterium glutamicum* ATCC13869 as a template, a homologous recombinant C arm was amplified using the primer pair of SEQ ID NOs: 21 and 22, and a homologous recombinant D arm was amplified using the primer pair of SEQ ID NOs: 23 and 24. Thereafter, a plasmid was obtained in the same manner as above, and this plasmid was named pDC24-argF(S55*).

Using the constructed pDC24-argR(E47*) vector, wild-type *Corynebacterium glutamicum* ATCC13869 was transformed by electroporation (Appl. Microbiol. Biotechnol. (1999) 52:541-545), and then through a second crossover process, a microorganism was constructed in which the 139th nucleotide sequence of argR was substituted from guanine (G) to thymine (T) and the 47th amino acid sequence was substituted with a stop codon. The corresponding genetic manipulation was validated by performing PCR and nucleotide sequence analysis using the primer pair of SEQ ID NOs: 17 and 20 which could amplify the adjacent region including gene insertion site. The microorganism obtained in this manner was named C.gl::argR*.

Microorganisms were constructed using the pDC24-argF(S55*) vector on the C.gl::argR*, in which the 164th nucleotide sequence of argF was substituted from cytosine (C) to adenine (A) and the 55th amino acid sequence was substituted with a stop codon. The corresponding genetic manipulation was validated by performing PCR and nucleotide sequence analysis using the primer pair of SEQ ID NOs. 21 and 24, which could amplify adjacent regions including the gene insertion site. The microorganism obtained in this manner was named C.gl::argR*_argF*.

The primer sequences used in Example 2-1 are shown in Table 2 below.

**[Table 2]**

| Name | Sequence (5'-> 3') | SEQ ID NO |
|---|---|---|
| Primer 1 | CGGTACCCGGGGATCCCTCGTGCGGAATTCG TGGAG | 17 |
| Primer 2 | ATCCAGCAGCAATTCAGACA | 18 |
| Primer 3 | CTGAATTGCTGCTGGATTAAGGCATCGATATC ACCCA | 19 |
| Primer 4 | ATGCCTGCAGGTCGACCCTTCATTTTAAGTTC CTTG | 20 |
| Primer 5 | CGGTACCCGGGGATCCTGACCCCAGGCAAG CACGG | 21 |
| Primer 6 | GAAGCGAGTACGAGTTTAAGTCTTATC | 22 |
| Primer 7 | AAACTCGTACTCGCTTCTCC | 23 |
| Primer 8 | ATGCCTGCAGGTCGACCGGCGCCGGCAACCT CGTC | 24 |

The vectors constructed in Example 1 were transformed into the C.gl::argR*_argF* strain constructed in Example 2-1 by electroporation to obtain strains introduced with the vectors.

Specifically, the strain introduced with pCES208-PbetP-argJ(C.gl) was named "C.gl::argR*_argF*-argJ(C.gl)", the strain introduced with pCES208-PbetP-argJ(T.ne) was named "C.gl::argR*_argF*-argJ(T.ne)", and the strain introduced with pCES208-PbetP-argJ(T.ma) was named "C.gl::argR*_argF*-argJ(T.ma)".

### 2-2. Evaluation of L-ornithine production ability of a microorganism introduced with the bifunctional glutamate n-acetyltransferase/amino acid acetyltransferase derived from Thermotoga maritima

In order to analyze L-ornithine production ability, the control strain C.gl::argR*_argF*-argJ(C.gl) strain, the *Corynebacterium glutamicum* C.gl::argR*_argF*-argJ(T.ne) strain, and the C.gl::argR*_argF*-argJ(T.ma) strain were cultured in the following manner, and OD₅₆₂, L-ornithine production amount, and L-ornithine production yield were measured.

Specifically, each strain was inoculated into a 250 ml corner-baffle flask containing 25 mL of the following production medium, and shaking culture was performed at 33 °C for 48 hours at 200 rpm. After completion of the culture, the culture medium was diluted 100-fold in a 0.1N HCl solution, OD₅₆₂ was measured with a spectrophotometer, and L-ornithine production amount and L-ornithine yield were measured using HPLC. The yield was calculated as a ratio (%) obtained by dividing the production amount by the consumed sugar as in Equation 1 below. $Yield \left(%\right) = \left(Production amount of product \left(g/L\right)/Consumed sugar \left(g/L\right)\right) \times 100$

### <Production medium (pH 7.2)>

Raw sugar 50 g, (NH₄)₂SO₄ 40 g, yeast extract 1 g, KH2PO4 1.1 g, magnesium sulfate heptahydrate 1.2 g, L-arginine 0.2 g, biotin 1 mg, thiamine hydrochloride 5 mg, calcium pantothenate 5 mg, nicotinamide 15 mg, MnSO₄ 10 mg, FeSO₄ 10 mg, ZnSO₄ 0.5 mg, CuSO₄ 0.5 mg, CaCO₃ 30 g, kanamycin 25 mg (based on 1 liter of distilled water)

The experiment was repeated 3 times, to show the average value of the analysis results in Table 3 below.

**[Table 3]**

| Strain | OD₅₆₂ | Consumed sugar (g/L) | L-ornithine production amount (g/L) | L-ornithine yield (%) |
|---|---|---|---|---|
| C.gl::*argR**_*argF**-*argJ*(C.gl) | 36.8 | 50.0 | 15.6 | 31.2 |
| C.gl::*argR**_*argF**-*argJ*(T.ne) | 38.1 | 50.0 | 15.7 | 31.6 |
| C.gl::*argR**_*argF**-*argJ*(T.ma) | 38.8 | 50.0 | 16.7 | 33.4 |

As shown in the results of Table 3 above, it was demonstrated that the L-ornithine productivity was increased by 7% on average in the C.gl::argR*_argF*-argJ(T.ma) strain expressing the bifunctional glutamate N-acetyltransferase/amino-acid acetyltransferase derived from *Thermotoga maritima* compared to the C.gl::argR*_argF*-argJ(C.gl) strain with enhanced argJ derived from *Corynebacterium*.

From the above results, it was demonstrated that the introduction of the argJ gene encoding the bifunctional glutamate N-acetyltransferase/amino-acid acetyltransferase derived from *Thermotoga maritima* increased the L-ornithine production ability of *Corynebacterium glutamicum*.

### Example 3. Evaluation of L-citrulline production ability of a microorganism introduced with the bifunctional glutamate N-acetyltransferase/amino acid acetyltransferase gene derived from a heat-resistant microorganism

### 3-1. Construction of microorganism producing L-citrulline

In order to construct an L-citrulline producing microorganism, a vector was constructed to replace phenylalanine at position 68 of the protein sequence of ArgG (ANU33620.1) with a stop codon.

By using the genome of wild-type *Corynebacterium glutamicum* ATCC13869 as a template, a homologous recombinant A arm was amplified using the primer pair of SEQ ID NOs: 25 and 26, and a homologous recombinant B arm was amplified using the primer pair of SEQ ID NOs: 27 and 28. Thereafter, a plasmid was obtained in the same manner as in Example 2-1, and this plasmid was named pDC24-argG(F68*).

In order to produce a microorganism with further improved productivity for L-citrulline from C.gl::argR* constructed in Example 2-1, a microorganism was constructed using the pDC24-argG(F68*) vector, in which the 203rd nucleotide sequence of argG was substituted from thymine (T) to adenine (A), and the 204th nucleotide sequence was substituted from cytosine (C) to adenine (T), so that the 68th amino acid sequence was substituted with a stop codon. The corresponding genetic manipulation was validated by performing PCR and nucleotide sequence analysis using the primer pair of SEQ ID NOs: 25 and 28 which could amplify the adjacent region including the gene insertion site. The microorganism obtained in this manner was named C.gl::argR*_argG*.

The primer sequences used in Example 3-1 are shown in Table 4 below.

**[Table 4]**

| Name | Sequence (5'-> 3') | SEQ ID NO: |
|---|---|---|
| Primer 9 | CGGTACCCGGGGATCCTTCATCGATAGGGTGGG | 25 |
| Primer 10 | GTACTCCTCAGCTTACTCATCCTTTGCATCAACA | 26 |
| Primer 11 | AGTAAGCTGAGGAGTACTGCCTGCCAACCATCAA | 27 |
| Primer 12 | ATGCCTGCAGGTCGACCGACTGGCTTGCCACCCT | 28 |

The vectors constructed above were transformed into the C.gl::argR*_argG* strain constructed in Example 3-1 by electroporation to obtain strains introduced with the vector.

Specifically, the strain introduced with pCES208-PbetP-argJ was named "C.gl::argR*_argG*-argJ(C.gl)", the strain introduced with pCES208-PbetP-argJ(T.ne) was named "C.gl::argR*_argG*-argJ(T.ne)", and the strain introduced with pCES208-PbetP-argJ(T.ma) was named "C.gl::argR*_argG*-argJ(T.ma)".

### Example 3-2: Evaluation of increased L-citrulline production ability of a microorganism introduced with the bifunctional glutamate n-acetyltransferase/amino acid acetyltransferase derived from Thermotoga maritima

In order to analyze L-citrulline production ability, the control strain *Corynebacterium glutamicum* C.gl::argR*_argG*-argJ(C.gl), the *Corynebacterium glutamicum* C.gl::argR*_argG*-argJ(T.ne) strain, and the C.gl::argR*_argG*-argJ(T.ma) strain were cultured in the following manner, and OD₅₆₂, L-citrulline production amount, and L-citrulline production yield were measured.

Specifically, each strain was inoculated into a 250 ml corner-baffle flask containing 25 ml of the following production medium, and shaking culture was performed at 33 °C for 48 hours at 200 rpm. After completion of the culture, OD₅₆₂ of the culture medium was measured, and L-citrulline production amount and L-citrulline yield were measured using HPLC in the same manner as in Example 2-2.

### <Production medium (pH 7.2)>

Raw sugar 50 g, (NH₄) ₂SO₄ 40 g, yeast extract 1 g, KH₂PO₄ 1.1 g, magnesium sulfate heptahydrate 1.2 g, L-arginine 0.2 g, biotin 1 mg, thiamine hydrochloride 5 mg, calcium pantothenate 5 mg, nicotinamide 15 mg, MnSO₄ 10 mg, FeSO₄ 10 mg, ZnSO₄ 0.5 mg, CuSO₄ 0.5 mg, CaCO₃ 30 g, kanamycin 25 mg (based on 1 liter of distilled water)

The experiment was repeated 3 times, to show the average value of the analysis results in Table 5 below.

**[Table 5]**

| Strain | OD₅₆₂ | Consumed sugar (g/L) | L-citrulline production amount (g/L) | L-citrulline yield (%) |
|---|---|---|---|---|
| C.gl::*argR**_*argG**-*argJ*(C.gl) | 44.4 | 50.0 | 4.8 | 9.6 |
| C.gl::*argR**_*argG**-*argJ*(T.ne) | 46.2 | 50.0 | 4.6 | 9.1 |
| C.gl::*argR**_*argG**-*argJ*(T.ma) | 47.3 | 50.0 | 5.8 | 11.5 |

As shown in the results of Table 5 above, it was demonstrated that the L-citrulline production ability was increased by 21% on average in the C.gl::argR*_argG*-argJ(T.ma) strain expressing the bifunctional glutamate N-acetyltransferase/amino-acid acetyltransferase derived from *Thermotoga maritima* compared to the C.gl::argR*_argG*-argJ(C.gl) strain with enhanced argJ derived from *Corynebacterium*.

From the above results, it was demonstrated that the introduction of the argJ gene encoding the bifunctional glutamate N-acetyltransferase/amino-acid acetyltransferase derived from *Thermotoga maritima* increased the L-citrulline production ability of *Corynebacterium glutamicum*.

### Example 4. Evaluation of putrescin production ability of a microorganism introduced with the bifunctional glutamate N-acetyltransferase/amino acid acetyltransferase gene derived from a heat-resistant microorganism

### 4-1. Construction of microorganism producing putrescin

In order to construct a putrescine producing microorganism, information on a gene encoding ornithine decarboxylase derived from Lactobacillus sp. 30A strain and surrounding nucleotide sequences was obtained from NIH GenBank (SEQ ID NO: 29).

Gene fragments for constructing a vector were obtained through PCR using sequences obtained through gene synthesis as templates based on the sequences obtained above.

SolgTM Pfu-X DNA polymerase was used as a polymerase, and PCR amplification conditions were as follows: initial denaturation at 95 °C for 5 minutes, followed by 30 cycles of denaturation at 95 °C for 30 seconds, annealing at 55 °C for 30 seconds, and extension at 72°C for 2 minute, with a final extension performed at 72 °C for 5 minutes.

More specifically, the primers of SEQ ID NOs: 31 and 32 were prepared to amplify the LODC (odci) gene derived from Lactobacillus sp. 30A strain, and PCR using the nucleotide sequence of SEQ ID NO: 30 as a template was performed to obtain a gene fragment of 2196 bp.

In order to obtain an expression promoter in the vector, PCR was performed in the same manner as above using wild-type *Corynebacterium glutamicum* ATCC13869 as a template and using primers of SEQ ID NO: 33 and SEQ ID NO: 34, to obtain a gene fragment of promoter region of 318 bp.

In order to delete NCgl1469 (SEQ ID NO: 41), by using the genome of wild-type *Corynebacterium glutamicum* ATCC13869 as a template, a homologous recombinant A arm was amplified using the primer pair of SEQ ID NOs: 35 and 36, and a homologous recombinant B arm was amplified using the primer pair of SEQ ID NOs: 37 and 38. PCR conditions were as follows: initial denaturation at 95°C for 10 minutes, followed by 30 cycles of denaturation at 95°C for 30 seconds, annealing at 55°C for 30 seconds, and extension at 72°C for 1 minute for 30 times, with a final extension performed at 72°C for 5 minutes.

A deletion vector was obtained by connecting the amplified promoter region, the gene fragments derived from Lactobacillus sp. 30A strain, and the homologous recombinant arms with the vector pDC24 digested with BamHI and XbaI restriction enzymes using an In-fusion Cloning Kit. This plasmid was named pDC24-ΔNCgl1469::Pcj7_LODC.

The sequences of the primers used in Example 4-1 are shown in Table 6 below.

**[Table 6]**

| Name | Sequence(5'-> 3') | SEQ ID NO |
|---|---|---|
| LODC_F | GAAAGGAAACACTCATGTCCTCTTCACTTA AAATTGC | 31 |
| LODC_R | CTTAAATCGCCTTCAGGGTTAATTATTATACC GGTCGTC | 32 |
| Pcj7_F | CGCTATTCTGGTATCCAGAAACATCCCAGCG C | 33 |
| Pcj7_R | TTTTAAGTGAAGAGGACATGAGTGTTTCCT TTCGTTGG | 34 |
| Primer 13 | AGCTCGGTACCCGGGGATCCCAGGAATACA GCTGTTC | 35 |
| Primer 14 | CTGGGATGTTTCTGGATACCAGAATAGCGA AATG | 36 |
| Primer 15 | GACGACCGGTATAATAATTAACCCTGAAGG CGATTTAAG | 37 |
| Primer 16 | GCCTGCAGGTCGACTCTAGAAATGGCAGAG TTGG | 38 |

The vectors constructed above were transformed into the C.gl::argR*_argF*-ΔNCgl1469::Pcj7_LODC strain constructed in Example 4-1 by electroporation to obtain strains introduced with the vector.

Specifically, the strain introduced with pCES208-PbetP-argJ was named "C.gl::argR*_argF*- ΔNCgl1469::Pcj7_LODC-argJ(C.gl)", the strain introduced with pCES208-PbetP-argJ(T.ne) was named "C.gl::argR*_argF*-ΔNCgl1469::Pcj7_LODC-argJ(T.ne)", and the strain introduced with pCES208-PbetP-argJ(T.ma) was named "C.gl::argR*_argF*-ΔNCgl1469::Pcj7_LODC-argJ(T.ma)".

### Example 4-2. Evaluation of increased putrescine production ability of a microorganism introduced with the bifunctional glutamate n-acetyltransferase/amino acid acetyltransferase derived from Thermotoga maritima

In order to analyze putrescine production ability, the control strain C.gl::argR*_argF*-ΔNCgl1469::Pcj7_LODC-argJ(C.gl), the C.gl::argR*_argF*-ΔNCgl1469::Pcj7_LODC-argJ(T.ne) strain, and the C.gl::argR*_argF*-ΔNCgl1469::Pcj7_LODC-argJ(T.ma) strain were cultured in the following manner, and OD₅₆₂, putrescine production amount, and putrescine yield were measured.

Specifically, each strain was inoculated into a 300 ml corner-baffle flask containing 25 ml of the following production medium, and shaking culture was performed at 33 °C for 47 hours at 200 rpm. After completion of the culture, putrescine production amount and putrescine yield were measured using HPLC in the same manner as in Example 2-2.

### <Production medium (pH 7.2)>

Glucose 60 g, CM (cane molasses) raw liquid 20 g, (NH₄)₂SO₄ 50 g, KH₂PO₄ 0.72 g, magnesium sulfate heptahydrate 1.13 g, MgCl₂ 0.4 g, MnSO4 0.18 g, ZnSO₄ 0.9 mg, CuSO₄ 0.9 mg, FeSO₄ 0.18 g, biotin 0.9 mg, thiamine hydrochloride 9 mg, calcium pantothenate 9 mg, nicotinamide 60 mg, L-arginine 0.15 g, corn steep liquor 8.12 g, CaCO₃ 50 g, kanamycin 25 mg (based on 1 liter of distilled water)

The experiment was repeated 3 times, to show the average value of the analysis results in Table 7.

**[Table 7]**

| Strain | OD₅₆₂ | consumed sugar (g/L) | putrescine production amount (g/L) | putrescine yield (%) |
|---|---|---|---|---|
| C*.*gl::*argR**_*argF**-Δ*NCgl1469*::Pcj7_*LODC* -*argJ*(C.gl) | 44.9 | 47.8 | 3.6 | 7.5 |
| C*.*gl::*argR**_*argF**-Δ*NCgl1469*::Pcj7_*LODC* -*argJ*(T.ne) | 42.3 | 40.0 | 1.8 | 4.6 |
| C*.*gl::*argR**_*argF**-Δ*NCgl1469*::Pcj7_*LODC* -*argJ*(T.ma) | 33.4 | 60.0 | 11.1 | 18.5 |

As shown in the results of Table 7, it was demonstrated that the putrescine productivity was increased by an average of 1.5 times in the C.gl::argR*_argF*-ΔNCgl1469::Pcj7_LODC-argJ(T.ma) strain expressing the bifunctional glutamate N-acetyltransferase/amino-acid acetyltransferase derived from *Thermotoga maritima* compared to the C.gl::argR*_argF*-ΔNCgl1469::Pcj7_LODC-argJ(C.gl) strain with enhanced argJ derived from *Corynebacterium*.

From the above results, it was demonstrated that the introduction of the argJ gene encoding the bifunctional glutamate N-acetyltransferase/amino-acid acetyltransferase derived from *Thermotoga maritima* increased the putrescine productivity of *Corynebacterium glutamicum*.

From the above description, those of ordinary skill in the art to which the present disclosure belongs will be able to understand that the present disclosure can be implemented in other specific forms without changing its technical spirit or essential features. In this regard, it should be understood that the examples described above are illustrative in all respects and not restrictive. The scope of the present disclosure should be interpreted as comprising all changes or modified forms derived from the meaning and scope of the following claims and their equivalent concepts rather than the detailed description.

## Claims

1. A microorganism of the genus *Corynebacterium*, expressing bifunctional glutamate N-acetyltransferase/amino-acid acetyltransferase derived from *Thermotoga maritima*.

2. The microorganism of the genus *Corynebacterium* according to claim 1, wherein the bifunctional glutamate N-acetyltransferase/amino-acid acetyltransferase derived from *Thermotoga maritima* comprises an amino acid sequence of SEQ ID NO: 1 or an amino acid sequence having sequence identity of 90% or more therewith.

3. The microorganism of the genus *Corynebacterium* according to claim 1, wherein the microorganism of the genus *Corynebacterium* is introduced by a polynucleotide encoding the bifunctional glutamate N-acetyltransferase/amino acid acetyltransferase.

4. The microorganism of the genus *Corynebacterium* according to claim 3, wherein the polynucleotide consists of a nucleotide sequence of SEQ ID NO: 2.

5. The microorganism of the genus *Corynebacterium* according to any one of claims 1 to 4, wherein the microorganism of the genus *Corynebacterium* has increased production ability of glutamate-family amino acid compared to a microorganism of the genus *Corynebacterium* that does not express the bifunctional glutamate N-acetyltransferase/amino acid acetyltransferase.

6. The microorganism of the genus *Corynebacterium* according to claim 5, wherein the glutamate-family amino acid is any one selected from the group consisting of L-ornithine, L-citrulline, L-arginine, and putrescine.

7. The microorganism of the genus *Corynebacterium* according to any one of claims 1 to 4, wherein the microorganism of the genus *Corynebacterium* is *Corynebacterium glutamicum*.

8. A method for producing a glutamate-family amino acid, comprising:
culturing the microorganism of the genus *Corynebacterium* according to any one of claims 1 to 4 in a culture medium; and
recovering the glutamate-family amino acid from the cultured microorganism, the medium, or both.

9. The method for producing a glutamate-family amino acid according to claim 8, wherein the glutamate-family amino acid is any one selected from the group consisting of L-ornithine, L-citrulline, L-arginine, and putrescine.

10. The method for producing a glutamate-family amino acid according to claim 8, wherein the microorganism of the genus *Corynebacterium* is *Corynebacterium glutamicum*.

11. Use of a microorganism of the genus *Corynebacterium* according to any one of claims 1 to 4 for producing glutamate-family amino acid.

12. A composition, method, product, process, or use **characterized by** one or more elements disclosed in the present disclosure.
